## Europäisches Patentamt
## European Patent Office
## Office européen des brevets

(19)

(11) Numéro de publication: **0 095 396**
**B1**

(12)

# FASCICULE DE BREVET EUROPEEN

(45) Date de publication du fascicule du brevet:
**27.08.86**

(51) Int. Cl.⁴: **A 61 F 5/01**

(21) Numéro de dépôt: **83400884.9**

(22) Date de dépôt: **03.05.83**

(54) Appareillage externe de station verticale et de marche pour handicapés moteurs des membres inférieurs.

(30) Priorité: **21.05.82 FR 8208892**

(43) Date de publication de la demande:
**30.11.83 Bulletin 83/48**

(45) Mention de la délivrance du brevet:
**27.08.86 Bulletin 86/35**

(84) Etats contractants désignés:
**AT BE CH DE GB IT LI LU NL SE**

(56) Documents cités:
**GB - A - 1 298 930
US - A - 1 336 695
US - A - 1 660 721
US - A - 2 654 365
US - A - 2 772 674
US - A - 3 805 773
US - A - 4 102 337**

**MEDIZINISCH-ORTHOPAEDISCHE TECHNIK, vol. 101,
no. 2, mars-avril 1981, pages 39-41, Stuttgart, DE. R.O.
NITSCHKE: "Laterale Hüft-Knie-Fuss-Orthesen"**

(73) Titulaire: **Salort, Guy, 219, rue Raymond Losserand,
F-75014 Paris (FR)**

(72) Inventeur: **Salort, Guy, 219, rue Raymond Losserand,
F-75014 Paris (FR)**

(74) Mandataire: **Peuscet, Jacques, 3, Square de Maubeuge,
F-75009 Paris (FR)**

## Description

Le brevet européen n° 0 066 028 qui est compris dans l'état de la technique au sens de l'article 54(3) de la CBE, a trait à un appareillage externe autonome qui permet la station verticale et la locomotion des handicapés moteurs d'un ou des deux membres inférieurs, cet appareillage étant constitué par un para-squelette amovible et léger permettant à la fois de pallier l'insuffisance du tonus postural et de reconstituer le mécanisme physiologique de l'appui podal et de la marche.

Ce para-squelette comporte:

a) un corset monocoque à hauteur de la ceinture pelvienne, suffisamment développé en hauteur pour rendre solidaire le centre de gravité corporel et les membres inférieurs;

b) parallèlement à la face externe du fémur et sensiblement sur sa longueur, un élément métallique, léger et flexible, susceptible d'encaisser et de restituer les contraintes de flexion et de torsion pour former un levier-ressort fémoral externe;

c) un levier-ressort métallique, léger et flexible, susceptible d'encaisser et de restituer les contraintes de flexion et de torsion et disposé parallèlement à la face antéro-interne du tibia, sensiblement sur toute sa longueur;

d) une sangle latérale verticale élastique reliant ledit ressort fémoral externe audit corset, et des sangles élastiques transversales se croisant en X, une pour chaque levier-ressort fémoral reliant chacune un ressort fémoral audit corset;

e) au niveau du genou, un ensemble d'éléments muni de sangles élastiques limitant le flexum, le valgum, le récurvatum et la rotation interne dans les limites étroites du jeu articulaire physiologique;

f) une chaussure, notamment orthopédique, reliée audit levier-ressort tibial pour le positionnement de l'articulaton tibio-tarsienne dans les limites d'un jeu physiologique normal.

Cet appareillage réalise, en fait, une véritable inversion du système physiologique du membre inférieur et, grâce à lui, une marche automatique devient possible par le biais du déplacement volontaire du centre de gravité du corps en arrière et latéralement, à la façon d'un «moteur pelvien».

Le corset monocoque, peu ou pas déformable, assure une semi-rigidité de la partie inférieure du tronc et permet d'assurer le mouvement moteur par le déplacement de la partie du tronc située au-dessus du corset, ce dernier ayant également pour rôle d'améliorer la liaison entre la partie d'appareillage d'étendant parallèlement au fémur et le bassin, notamment au niveau des ailes iliaques.

La genouillère, qui est capable de maintenir l'articulation du genou en léger flexum et léger valgum, au moyen d'un système de sangles élastiques convenablement disposées pour former des freins élastiques au récurvatum, au varum, au valgum et au flexum, est également conçue pour constituer un frein aux rotations interne et externe et pour avoir tendance à favoriser la rotation interne stabilisant ainsi la tête fémorale pour assurer une recentrage à chaque pas, le membre inférieur étant, en fait, sollicité en vis interne lors de son mouvement.

Le déposant, pour améliorer le fonctionnement de l'appareillage selon le brevet européen 0 066 028, a mis au point une chaussure orthopédique qui permet le fonctionnement physiologique de l'appui podal en confortant et en régulant un mouvement de vis interne.

La présente invention a donc pour objet le produit industriel nouveau que constitue un appareillage externe de station verticale et de marche pour handicapés moteurs d'un (ou des deux) membre(s) inférieur(s), ledit appareillage comportant, en combinaison:

– un corset monocoque disposé à hauteur de la ceinture pelvienne;

– une armature associée à chaque membre à appareiller, ladite armature comportant:

– d'une part, un élément fémoral externe disposé parallèlement à la face externe du fémur et sensiblement sur toute sa longueur, ledit élément ayant son extrémité supérieure reliée au corset, au voisinage de la crête iliaque, par un moyen, qui est disposé sensiblement dans le prolongement de l'élément et qui est fixé audit corset;

– et, d'autre part, un élément tibial, disposé du côté de la face interne du tibia et sensiblement sur toute sa longueur, ledit élément étant relié à l'élément fémoral, au niveau du genou, par un organe de liaison permettant leurs mouvement relatifs;

– l'élément fémoral externe étant un levier-ressort comprenant une lame de métal flexible susceptible d'encaisser et restituer les contraintes de flexion et de torsion;

– l'élément tibial étant un levier-ressort antéro-interne comprenant une lame de métal flexible susceptible d'encaisser et de restituer les contraintes de flexion et de torsion;

– l'organe de liaison étant une genouillère susceptible de maintenir les mouvements relatifs entre la cuisse et la jambe en léger flexum et léger valgum, dans les limites étroites du jeu articulaire physiologique, grâce à des éléments élastiques disposés, du côté interne et du côté externe du genou, entre des zones de la genouillère situées au-dessus et au-dessous de l'articulation, tant sur la face frontale que sur la face postérieure;

– le moyen, qui relie le levier-ressort fémoral au corset, étant une première sangle élastique, ledit levier-ressort fémoral étant, en outre, relié au corset par une deuxième sangle élastique, qui s'étend transversalement en étant légèrement inclinée vers le haut pour se fixer sur le corset vers ou en direction des épines iliaques, ladite deuxième sangle croisant ainsi sensiblement la symphyse publienne;

– la genouillère étant reliée rigidement, d'une part, dans sa zone supérieure, à l'extrémité inférieure du levier-ressort fémoral et, d'autre part, dans sa zone inférieure, à l'extrémité supérieure du levier-ressort tibial;

– l'extrémité inférieure du levier-ressort tibial étant reliée rigidement à un élément, qui limite le jeu articulaire de l'articulation tibio-tarsienne, ledit élément étant une chaussure orthopédique qui comprend:

– une coque semi-rigide entourant le pied et le cône inférieur jambier, cette coque comportant une ouverture, qui s'étend en regard de la loge antéro-externe du cône jambier et en regard du bord externe du pied, au-dessus du cuboïde et d'au moins un rayon métatarsien, ladite coque portant dans la zone de la malléole interne un point d'attache de l'extrémité inférieure du levier-ressort tibial;

– une armature élastique comportant d'une part, un levier-ressort lié à la coque et disposé verticalement en regard du péroné, depuis la face externe du calcanéum jusqu'à la zone de bordure supérieure de la coque, et, d'autre part, des moyens élastiques régulateurs implantés à l'extérieur de la coque, en premier lieu, à partir du bord interne de la semelle de la coque vers la partie supérieure de l'empeigne et vers la bordure supérieure de la tige de la coque, et en second lieu, à partir du point d'attache du levier-ressort tibial jusqu'à la zone postéro-externe du talon de la semelle;

– et des moyens de fermeture de la coque de la chaussure.

Avantageusement, les moyens de fermeture de la coque comprennent un moyen élastique tendu entre le bord interne de la semelle de la coque, au niveau du premier métatarsien, et le bord externe de ladite semelle au niveau du cuboïde; éventuellement, les moyens de fermeture de la coque peuvent comprendre un moyen élastique tendu entre le bord interne de la semelle de la coque, au niveau du premier métatarsien, et le bord externe de ladite semelle au niveau du cinquième métatarsien; en bout du pied, l'ouverture de la coque s'étend transversalement au-dessus des deux derniers métatarsiens; la coque entoure le cône inférieur jambier jusqu'au dessus du tiers inférieur de la jambe et les moyens de fermeture comprennent une bande de serrage s'étendant tout le long de la bordure supérieure de la tige de la coque; la coque de la chaussure peut être réalisée d'une seule pièce en matière plastique moulée; la chaussure peut comprendre intérieurement une semelle de soutien du pied moulée aux reliefs de la voûte plantaire de l'utilisateur mis en position d'équilibre vertical.

Le levier-ressort, lié à la coque de la chaussure et disposé verticalement en regard du péroné, peut être une lame d'acier trempé de 40 mm de large et de 1 mm d'épaisseur environ: ce levier-ressort régule les mouvements d'inclinaison interne (roulement et abaissement du calcanéum) et les mouvements de vis interne du segment jambier sur les articulations tibio-tarsienne et sous-astragalienne. Les moyens élastiques régulateurs, qui sont associés à ce levier-ressort de la coque, régulent les actions du levier-ressort de la coque et du levier-ressort tibial interne. On a constaté que cette architecture particulière de la chaussure permet d'encaisser de légères déformations en mouvement de vis interne et de restituer élastiquement les efforts: il en résulte une légère flexion physiologique de la cheville ainsi qu'un déroulement du pas podal (talon-bord externe-bord interne).

La présente demande précise, en outre, un certain nombre de particularités interessant les divers éléments de structure de l'appareillage décrit dans le brevet européen 0 066 028.

Ainsi, les deux sangles élastiques reliant l'extrémité supérieure du levier fémoral externe et le corset, respectivement, au voisinage de la crête iliaque et en direction des épines iliaques, peuvent être de longueur réglable.

De même, de façon avantageuse, le levier-ressort fémoral externe s'étend entre une extrémité supérieure sous-trochantérienne et une extrémité inférieure disposée sensiblement au-dessus du condyle fémoral, et le levier-ressort tibial, en position antéro-interne, s'étend de façon avantageuse entre une extrémité supérieure disposée sous le plateau tibial et une extrémité inférieure sus-malléolaire. Une précontrainte appropriée, par exemple de quelques déci-Newtons, peut être appliquée aux leviers-ressorts, selon l'état du tonus postural du sujet.

La genouillère forme, de préférence, une seule pièce souple constituée de trois parties: un cône fémoral inférieur, un cône tibial supérieur et un cône para-rotulien entre les deux précédents. On pourrait également envisager de réaliser cette genouillère en deux ou trois éléments distincts reliés entre eux par des moyens appropriés, de préférence élastiques.

En outre, comme indiqué dans le brevet européen 0 066 028, la genouillère comporte une pluralité de sangles constituant des freins au récurvatum, varum, valgum, flexum, ainsi qu'aux rotations interne et externe. Ces sangles sont, de préférence, réglables de façon à adapter la genouillère à chaque cas précis pour maintenir l'articulation normalement en léger flexum et léger valgum. Le flexum peut être, par exemple de l'ordre de 120 à 160° et, de façon avantageuse, être tel qu'à l'état de repos, lorsque le sujet est debout, le plateau tibial forme avec le plan horizontal un angle vers le bas et vers l'avant qui ne soit pas inférieur à 5°. De même, l'angle du plateau en valgum est, de préférence, égal ou supérieur à 5° par rapport à l'horizontale.

A titre d'exemple, pour un sujet de 40 kg, la tension des sangles élastiques de l'appareillage pourra être voisine de 0,6 Newton.

Les sangles élastiques de l'appareillage ou les bandes élastiques des chaussures dudit appareillage peuvent être non seulement constituées de tissus élastiques ou de bandes de caoutchouc, mais elles peuvent également être constituées de tout élément susceptible d'exercer une tension élastique entre les deux points d'accrochage de ces sangles ou bandes; on pourrait envisager de les réaliser en utilisant des parties non élastiquement déformables reliées entre elles par un élément susceptible d'assurer une régulation de la tension entre ces parties, par exemple par un moyen électronique ou mécanique approprié.

Un autre invention, ayant trait au même sujet, fait l'objet de la demande divisionnaire no 851 010 322 (numéro de publication: EP-A 0 160 780; date de la publication: 13.11.85).

D'autres avantages et caractéristiques de l'invention apparaîtront à la lecture de la description suivante, faite à titre d'exemple non limitatif en se référant au dessin annexé.

Sur ce dessin:

– la fig. 1 représente, en perspective, le détail d'une chaussure de l'appareillage selon l'invention, vue du côté interne;

– la fig. 2 représente, en perspective, la chaussure de la fig. 1 vue du côté externe.

Sur le dessin, on a représenté le levier-ressort tibial 6 de l'appareillage selon l'invention. Ce levier-ressort 6, qui est inséré sur la genouillère en position antéro-interne, s'étend le long de la diaphyse tibiale jusqu'en son extrémité inférieure 6b, au niveau de la malléole interne, où il est fixé sur la tige montante de la chaussure orthopédique 7.

La chaussure 7 est constituée d'une coque 70 en matière plastique moulée d'une épaisseur de 3 mm, par exemple en polyéthylène basse densité (d = 0,95). Cette coque, réalisée d'une seule pièce, est constituée d'une semelle 701 surmontée par une empeigne, dont la partie supérieure a été désignée par 703 et par une tige, dont la bordure supérieure 702 est destinée à se trouver légèrement au-dessus du tiers inférieur de la jambe. La tige et l'empeigne de la coque semi-rigide 70 comportent une ouverture 71, qui s'étend verticalement sur la tige en regard de la loge antéro-externe du cône jambier destiné à y être placé et qui s'étend sur l'empeigne en regard du bord externe du pied au-dessus du cuboïde; l'extrémité avant de cette ouverture 71 est disposée transversalement au-dessus des deux derniers rayons métatarsiens. La coque 70 porte dans la zone de la malléole interne, un point d'attache 72 destiné à fixer l'extrémité inférieure 6b du levier-ressort tibial 6 de l'apareillage selon l'invention. L'ouverture 71 permet un logement facile du pied et du cône inférieur jambier dans la coque 70. En outre, cette ouverture donne un degré de liberté, qui assure l'équilibre dynamique du mouvement du pied handicapé. A l'intérieur de la coque 70, sur la semelle 701, on dispose une semelle de soutien (non visible sur le dessin) moulée aux reliefs de la voûte plantaire du sujet en position d'équilibre vertical.

La chaussure selon l'invention comporte une armature insérée sur la coque et comprenant un levier-ressort 73 et des moyens élastiques régulateurs 74, 75 et 77.

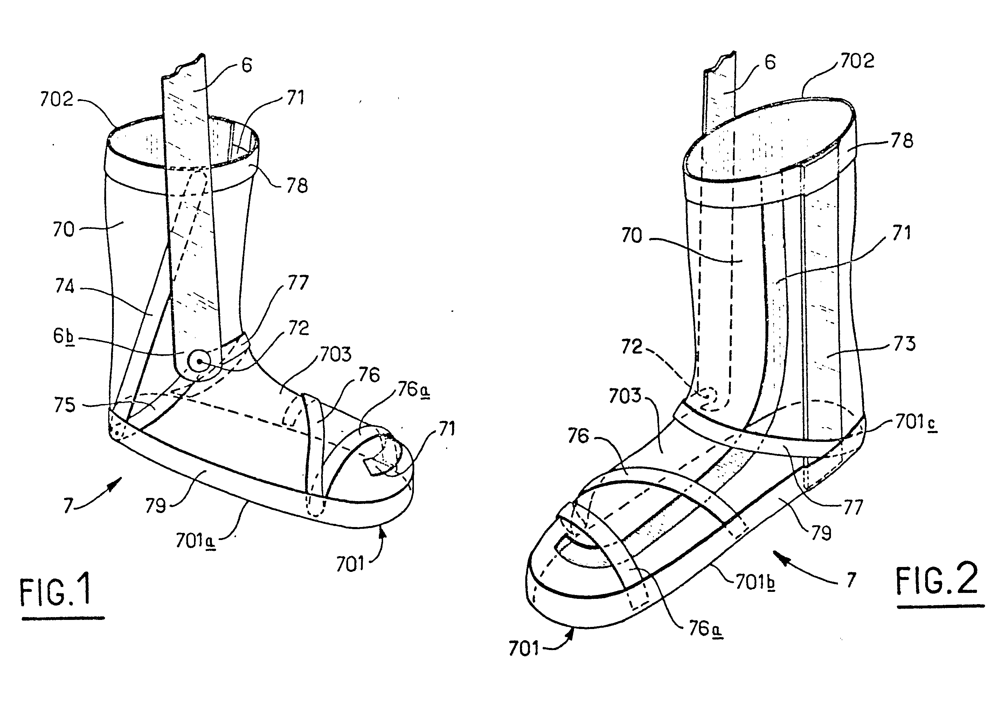

Le levier-ressort 73 est lié à la coque 70 au moins par ses deux extrémités. Dans la réalisation décrite, le levier-ressort 73 est collé extérieurement sur la coque 70 par ses deux extrémités; il est constitué d'une lame d'acier trempé de 40 mm de largeur sur 1 mm d'épaisseur. Dans une variante, on pourrait mettre en place ce levier-ressort en insert dans la coque 70 au moment du moulage de cette coque. Le levier-ressort 73 est disposé verticalement en regard du péroné depuis la face externe du calcanéum jusqu'à la zone de bordure supérieure 702 de la coque: il est visible sur la fig. 2.

Les moyens élastiques régulateurs sont constitués d'une pluralité de bandes élastiques, qui compensent et régulent les actions du levier-ressort 73 et du levier-ressort tibial 6. Une bande élastique 74 est tendue depuis un point situé au niveau de la semelle 701 dans le cadran postéro-interne du talon jusqu'à la bordure supérieure 702 de la tige de la coque 70. La bande 74 fait un angle d'environ 30° avec la verticale; elle constitue un frein élastique régulant les mouvements de la face interne de la chaussure et favorisant les mouvements de vis interne. Une bande élastique 75 est disposée à partir du même point de la bordure interne 701a de la semelle 701; elle s'étend jusqu'au point d'implantation 72 du levier-ressort tibial 6 et constitue un frein élastique régulant l'action du levier-ressort 6 sur la chaussure 7. Une bande élastique 77 est tendue entre le point d'attache 72 du levier-ressort 6 et un point situé dans la zone postéro-externe 701c de la bordure externe 701b de la semelle 701; cette bande élastique 77 agit également comme un moyen de fermeture de la chaussure, puisqu'elle passe pardessus l'ouverture 71 au niveau du coude-pied.

La chaussure 7 comporte, en outre, des moyens de fermeture, dont certains ont également un rôle de frein élastique au cours du mouvement. A cet égard, une bande élastique 76 est tendue entre un point situé sur la bordure interne 701a de la semelle 701, en regard du premier métatarsien, et un point situé sur la bordure externe 701b de la semelle 701, au niveau du bord externe du cuboïde; cette bande élastique 76 régule les mouvements de la partie supérieure 703 de l'empeigne de la chaussure et assure en même temps la fermeture de la chaussure, puisqu'elle est disposée transversalement par rapport à l'ouverture 71. On peut éventuellement, sans que cela soit obligatoire, mettre en place une autre bande élastique 76a, qui part du même point de la bordure interne 701a que la bande élastique 76 et qui va en un point de la bordure externe 701b de la semelle 701 situé en regard du cinquième métatarsien. La partie supérieure de la tige de la coque 70 est fermée par une bande de serrage 78 disposée tout le long de la bordure 702. Enfin, une bande 79 disposée tout autour de la semelle 701 enserre tous les points d'insertion de moyens élastiques disposés au niveau de la semelle 701. Toutes les bandes élastiques figurant dans la constitution de la chaussure 7 ont avantageusement une longueur réglable de façon à être ajustée aux caractéristiques du sujet, qui utilise l'appareillage.

On constate que l'utilisation d'une telle chaussure permet une légère flexion physiologique de la cheville au cours du déplacement du sujet ainsi qu'un déroulement du pas podal (talon-bord externe-bord interne).

**Revendications**

1. Appareillage externe de station verticale et de marche pour handicapés moteurs d'un (ou des deux) membre(s) inférieur(s), ledit appareillage comportant, en combinaison:
   – un corset monocoque disposé à hauteur de la ceinture pelvienne;
   – une armature associée à chaque membre à appareiller, ladite armature comportant:
   – d'une part, un élément fémoral externe disposé parallèlement à la face externe du fémur et sensiblement sur toute sa longueur, ledit élément ayant son extrémité supérieure reliée au corset, au voisinage de la crête iliaque, par un moyen, qui est disposé sensiblement dans le prolongement de l'élément et qui est fixé audit corset;
   – et, d'autre part, un élément tibial, disposé du côté de la face interne du tibia et sensiblement sur toute sa longueur, ledit élément étant relié à l'élément fémoral, au niveau du genou, par un organe de liaison permettant leurs mouvements relatifs;
   – l'élément fémoral externe étant un levier-ressort comprenant une lame de métal flexible susceptible d'encaisser et restituer les contraintes de flexion et de torsion;
   – l'élément tibial étant un levier-ressort antéro-interne comprenant une lame de métal flexible susceptible d'encaisser et de restituer les contraintes de flexion et de torsion;
   – l'organe de liaison étant une genouillère susceptible de maintenir les mouvements relatifs entre la cuisse et la jambe en léger flexum et léger valgum, dans les limites étroites du jeu articulaire physiologique, grâce à des éléments élastiques disposés, du côté interne et du côté externe du genou, entre des zones de la genouillère situées au-dessus et au-dessous de l'articulation, tant sur la face frontale que sur la face postérieure;
   – le moyen qui relie le levier-ressort fémoral au corset, étant une première sangle élastique, ledit levier-ressort fémoral étant, en outre, relié au corset par une deuxième sangle élastique, qui s'étend transversalement en étant légèrement inclinée vers le haut pour se fixer sur le corset vers ou en direction des épines iliaques, ladite deuxième sangle croisant ainsi sensiblement la symphyse pubienne;
   – la genouillère étant reliée rigidement, d'une part, dans sa zone supérieure, à l'extrémité inférieure du levier-ressort fémoral et, d'autre part, dans sa zone inférieure, à l'extrémité supérieure du levier-ressort tibial;
   – l'extrémité inférieure du levier-ressort tibial étant reliée rigidement à un élément, qui limite le jeu articulaire de l'articulation tibio-tarsienne, ledit élément (7) étant une chaussure orthopédique qui comprend:

   – une coque semi-rigide (70) entourant le pied et le cône inférieur jambier, cette coque comportant une ouverture (71), qui s'étend en regard de la loge antéro-externe du cône jambier et en regard du bord externe du pied, au-dessus du cuboïde et d'au moins un rayon métatarsien, ladite coque portant dans la zone de la malléole interne un point d'attache (72) de l'extrémité inférieure (6b) du levier-ressort tibial (6);
   – une armature élastique comportant d'une part, un levier-ressort (73) lié à la coque (70) et disposé verticalement en regard du péroné, depuis la face externe du calcanéum jusqu'à la zone de bordure supérieure (702) de la coque (70), et, d'autre part, des moyens élastiques régulateurs (74, 75, 77) implantés à l'extérieur de la coque, en premier lieu, à partir du bord interne (701a) de la semelle (701) de la coque (70) vers la partie supérieure (703) de l'empeigne et vers la bordure supérieure (702) de la tige de la coque (70), et en second lieu, à partir du point d'attache (72) du levier-ressort tibial (6) jusqu'à la zone postéro-externe (701c) du talon de la semelle (701);
   – et des moyens de fermeture (76, 76a, 78) de la coque (70) de la chaussure.

2. Appareillage selon la revendication 1, caractérisé par le fait que les moyens de fermeture de la coque (70) comprennent un moyen élastique (76) tendu entre le bord interne (701a) de la semelle (701) de la coque (70), au niveau du premier métatarsien, et le bord externe (701b) de ladite semelle, au niveau du cuboïde.

3. Appareillage selon l'une des revendications 1 ou 2, caractérisé par le fait que les moyens de fermeture de la coque (70) comprennent un moyen élastique (76a) tendu entre le bord interne (701a) de la semelle (701) de la coque (70), au niveau du premier métatarsien, et le bord externe (701b) de ladite semelle, au niveau du cinquième métatarsien.

4. Appareillage selon l'une des revendications 1 à 3, caractérisé par le fait qu'en bout du pied, l'ouverture (71) de la coque (70) s'étend transversalement au-dessus des deux derniers métatarsiens.

5. Appareillage selon l'une des revendications 1 à 4, caractérisé par le fait que la coque (70) entoure le cône inférieur jambier jusqu'au dessus du tiers inférieur de la jambe et que les moyens de fermeture comprennent une bande de serrage (78) s'étendant tout le long de la bordure supérieure (702) de la tige de la coque (70).

6. Appareillage selon l'une des revendications 1 à 5, caractérisé par le fait que la coque (70) de la chaussure (7) est réalisée d'une seule pièce en matière plastique moulée.

7. Appareillage selon l'une des revendications 1 à 6, caractérisé par le fait que la chaussure (7) comprend intérieurement une semelle de soutien du pied, moulée aux reliefs de la voûte plantaire de l'utilisateur mis en position d'équilibre vertical.

8. Appareillage selon l'une des revendications 1 à 7, caractérisé par le fait que le levier-ressort tibial (6) s'étend entre une extrémité supérieure sensiblement sous-articulaire et une extrémité inférieure sensiblement sus-malléolaire.

9. Appareillage selon l'une des revendications 1 à 8, caractérisé par le fait qu'une précontrainte appropriée est appliquée aux leviers-ressorts, selon l'état du tonus postural du sujet.

**Patentansprüche**

1. Äusserlich anbringbare Vorrichtung für Personen, bei denen ein oder beide untere(s) Gliedmass(e) motorisch behindert ist (sind), um diesen Personen senkrechtes Stehen und Gehen zu ermöglichen, wobei diese Vorrichtung in Kombination aufweist:
– ein einschaliges, in Höhe des Beckengürtels angebrachtes Korsett;
– eine mit jedem zusammenzupassenden Teil verbundene Bewehrung,
– die einerseits ein äusseres femorales Element, das parallel zur Aussenfläche des Femurs und im wesentlichen auf der ganzen Länge dazu angebracht ist, wobei dieses Element am oberen Ende in der Nähe des Hüftbeinkammes über ein Mittel, das im wesentlichen in der Verlängerung des Elementes angeordnet und am Korsett befestigt ist, mit dem Korsett verbunden ist,
– und andererseits ein tibiales Element aufweist, das neben der Innenfläche der Tibia und im wesentlichen auf der ganzen Länge dazu angebracht ist, wobei dieses Element mit dem femoralen Element in Kniehöhe über ein Verbindungsorgan verbunden ist, welches ermöglicht, dass sich diese Elemente relativ zueinander bewegen können, wobei
– das äussere femorale Element eine Hebel-Federkombination ist, die ein flexibles Metallblatt umfasst, welches die Beugungs- und Torsionsbeanspruchungen aufnehmen und wieder abgeben kann;
– das tibiale Element eine antero-innere Hebel-Federkombination ist, die ein flexibles Metallblatt aufweist, welches die Beugungs- und Torsionsbeanspruchungen aufnehmen und wieder abgeben kann;
– das Verbindungsorgan ein Knieband ist, das die Bewegungen des Oberschenkels relativ zum Unterschenkel in leichter flexus- und leichter valgus-Stellung in den engen Grenzen des physiologischen Gelenkspiels hält mit Hilfe von elastischen Elementen, welche an der Innenseite und der Aussenseite des Knies zwischen den Zonen des Gelenkbandes angeordnet sind, welche oberhalb und unterhalb des Gelenks sowohl auf der Frontseite als auch auf der rückwärtigen Seite angeordnet sind;
– das Mittel, welches die femorale Hebel-Federkombination mit dem Korsett verbindet, ein erster elastischer Gurt ist, wobei diese femorale Hebel-Federkombination ausserdem mit dem Korsett über einen zweiten elastischen Gurt verbunden ist, der sich transversal, wobei er leicht nach oben geneigt ist, um sich an dem Korsett zu fixieren, nach oder in Richtung der Darmbeinstachel erstreckt, wobei dieser zweite Gurt so im wesentlichen die Schambeinfuge kreuzt;
– das Knieband fest einerseits im oberen Bereich mit dem unteren Ende der femoralen Hebel-Federkombination und andererseits im oberen Bereich mit dem unteren Ende der tibialen Hebel-Federkombination verbunden ist; und
das untere Ende der tibialen Hebel-Federkombination fest mit einem Element verbunden ist, welches das Gelenkspiel des tibio-tarsalen Gelenks begrenzt, wobei dieses Element (7) ein orthopädischer Schuh ist, der aufweist:
– eine semi-steife Schale (70), welche den Fuss und den unteren Beinkonus umgibt, wobei diese Schale eine Öffnung (71) aufweist, die sich gegenüber der antero-externen Lage des Beinkonus und gegenüber dem Aussenrand des Fusses oberhalb des Würfelbeins und von mindestens einem metatarsalen Streifen erstreckt, wobei diese Schale im Bereich des Malleolus tibiae einen Befestigungspunkt (72) für das untere Ende (6b) der tibialen Hebel-Federkombination (6) aufweist;
– eine elastische Bewehrung, die einerseits eine Hebel-Federkombination (73), die mit der Schale (70) verbunden ist und vertikal gegenüber dem Wadenbein beginnend bei der Aussenfläche des Fersenbeins und endend im Bereich des oberen Randes (702) der Schale (70) angeordnet ist, und andererseits elastische Regulierungsmittel (74, 75, 77) aufweist, die aussen an der Schale erstens ausgehend vom Innenrand (701a) der Fusssohle (701) der Schale (70) zum oberen Teil (703) des Oberleders und zum oberen Rand (702) des Schafts der Schale (70) und zweitens ausgehend vom Befestigungspunkt (72) der tibialen Hebel-Federkombination (6) bis zum postero-äusseren Bereich (701c) der Ferse der Fusssohle (701) angeordnet sind;
– und Verschlussmittel (76, 76a, 78) für die Schale (70) des Schuhs.

2. Vorrichtung nach Anspruch 1, dadurch gekennzeichnet, dass die Verschlussmittel für die Schale (70) ein elastisches Mittel (76) aufweisen, das zwischen dem Innenrand (701a) der Fusssohle (701) der Schale (70) im Bereich des ersten Metatarsalknochens und dem Aussenrand (701b) dieser Fusssohle im Bereich des Würfelbeins gespannt ist.

3. Vorrichtung nach einem der Ansprüche 1 oder 2, dadurch gekennzeichnet, dass die Verschlussmittel für die Schale (70) ein elastisches Mittel (76a) aufweisen, das zwischen dem Innenrand (701a) der Fusssohle (701) der Schale (70) im Bereich des ersten Metatarsalknochens und dem Aussenrand (701b) der Fusssohle im Bereich des fünften Metatarsalknochens gespannt ist.

4. Vorrichtung nach einem der Ansprüche 1 bis 3, dadurch gekennzeichnet, dass sich die Öffnung (71) der Schale (70) am Fussende transversal oberhalb der beiden letzten Metatarsalknochen erstreckt.

5. Vorrichtung nach einem der Ansprüche 1 bis 4, dadurch gekennzeichnet, dass die Schale (70) den unteren Beinkonus bis oberhalb des unteren Beindrittels umgibt und dass die Verschlussmittel ein Zugband (78) umfassen, das sich entlang dem oberen Rand (702) des Schaftes der Schale (70) erstreckt.

6. Vorrichtung nach einem der Ansprüche 1 bis 5, dadurch gekennzeichnet, dass die Schale (70) des Schuhs (7) einstückig aus einem Plastikmaterial geformt ist.

7. Vorrichtung nach einem der Ansprüche 1 bis 6, dadurch gekennzeichnet, dass der Schuh (7) innen eine Fussstützsohle aufweist, die nach dem Fussgewölbe des in der vertikalen Gleichgewichtslage befindlichen Benutzers geformt ist.

8. Vorrichtung nach einem der Ansprüche 1 bis 7, dadurch gekennzeichnet, dass sich die tibiale Hebel-Federkombination (6), zwischen einem im wesentlichen unterartikulatorischen oberen Ende und einem im wesentlichen übermaleolaren unteren Ende erstreckt.

9. Vorrichtung nach einem der Ansprüche 1 bis 8, dadurch gekennzeichnet, dass die Hebel-Federkombination in geeigneter Weise je nach dem Zustand des Lagetonus des Patienten vorgespannt sind.

## Claims

1. An external apparatus for the vertical stance and walking for persons with motor handicaps in one or both lower limbs, the said apparatus comprising in combination:
- a single shell corset disposed at the level of the pelvic girdle;
- a brace associated with each limb to be equipped, the said brace comprising:
- on the one hand, an external femoral element disposed parallel to the external side of the femur and substantially over all its length, the said element having its upper end joined to the corset near the iliac crest by a means which is disposed substantially in the extension of the element and which is fixed to the said corset;
- and, on the other hand, a tibial element disposed on the internal side of the tibia and substantially over all its length, the said element being joined to the femoral element, at the level of the knee, by a connecting member allowing their relative movements; wherein
- the external femoral element is a spring lever comprising a strip of flexible metal capable of absorbing and restoring the flexural and torsional stresses;
- the tibial element is an antero-internal spring lever comprising a strip of flexible metal capable of absorbing and restoring the fexural and torsional stresses
- the connecting member is a knee cap capable of maintaining the relative movement between the thigh and leg in slight flexion and slight valgus, within the narrow limits of the physiological articular play, thanks to elastic elements disposed on the internal side and on the external side of the knee, between the zones of the knee cap situated above and below the articulation, both on its frontal side and on its posterior side;
- the means joining the femoral spring lever to the corset is a first elastic strap, the said femoral spring lever being additionally joined to the corset by a second elastic strap which extends transversely whilst being slightly inclined towards the top to be fixed on the corset towards or in the direction of the iliac spines, the said second strap thus substantially crossing the pubic symphisis;
- the knee cap is rigidly joined, on the one hand, in its upper zone, to the lower end of the femoral spring lever and, on the other hand, in its lower zone to the upper end of the tibial spring lever;
- the lower end of the tibial spring lever is rigidly joined to an element limiting the articular play of the tibial-tarsal articulation, the said element (7) being an orthopaedic shoe which comprises:
- a semi-rigid shell (70) surrounding the foot and the lower tibial conus, this shell comprising an opening (71) which extends opposite the antero-external loculus of the tibial conus and opposite the external edge of the foot, above the cuboid and at least one metatarsal zone, the said shell carrying in the zone of the internal malleolus an attachment point (72) for the lower end (6b) of the tibial spring lever (6);
- an elastic brace comprising on the one hand, a spring lever (73) joined to the shell (70) and disposed vertically opposite the fibula from the external side of the calcaneus as far as the zone of the upper edge (702) of the shell (70) and, on the other hand, regulating elastic means (74, 75, 77) fitted outside the shell firstly, from the internal edge (701a) of the sole (701) of the shell (70) towards the upper portion (703) of the vamp and towards the upper edge (702) of the trunk of shell (70) and secondly, from the attachment point (72) of the tibial spring lever (6), as far as the postero external zone (701c) of the heel of sole (701);
- and closing means (76, 76a, 78) for the shell (70) of the shoe.

2. An apparatus according to Claim 1, characterised in that the closing means for the shell (70) comprise an elastic means (76) stretched between the internal edge (701a) of the sole (701) of shell (70), at the level of the first metatarsal, and the external edge (701b) of the said sole, at the level of the cuboid.

3. An apparatus according to one of Claims 1 or 2, characterised in that the closing means for the shell (70) comprise an elastic means (76a) stretched between the internal edge (701a) of the sole (701) of the shell (70) at the level of the first metatarsal and the external edge (701b) of the said sole, at the level of the fifth metatarsal.

4. An apparatus according to one of Claims 1 to 3, characterised in that at the foot end, the opening (71) of the shell (70) extends transversely above the last two metatarsals.

5. An apparatus according to one of Claims 1 to 4, characterised in that the shell (70) surrounds the lower tibial conus as far as above the lower third of the leg and that the closing means comprise a tightening band (78) extending all along the upper edge (702) of the trunk of the shell (70).

**0 095 396**

6. An apparatus according to one of Claims 1 to 5, characterised in that the shell (70) of the shoe (7) is made integrally of a moulded plastic material.

7. An apparatus according to one of Claims 1 to 6, characterised in that the shoe (7) comprises internally a supporting sole for the foot, moulded to the contours of the plantar arch of the user placed in the position of vertical equilibrium.

8. An apparatus according to one of Claims 1 to 7, characterised in that the tibial spring lever (6) extends between an upper end substantially below the articulation and a lower end substantially above the malleolus.

9. An apparatus according to one of Claims 1 to 8, characterised in that an appropriate pre-stressing is applied to the spring levers according to the state of the subject's postural tonus.

FIG.1

FIG.2

0 095 396